# EUROPEAN PATENT SPECIFICATION

(11) **EP 3 474 798 B1**
(45) Date of publication and mention of the grant of the patent: **24.07.2024**
(21) Application number: 17734953.7
(22) Date of filing: 23.06.2017
(51) Int. Cl.: A61F 13/00, A61M 27/00

(54) **NEGATIVE PRESSURE WOUND THERAPY ARTICLE WITH PROTRUSIONS**
UNTERDRUCKWUNDTHERAPIEVORRICHTUNGSARTIKEL MIT PROTRUSIONEN
ARTICLE DE THÉRAPIE DE PLAIES PAR PRESSION NÉGATIVE DOTÉ DE PROTRUSIONS

(30) Priority: 27.06.2016 US 201662354894 P
(43) Date of publication of application: 01.05.2019
(73) Proprietor: Solventum Intellectual Properties Company, Maplewood, MN 55144 (US)
(72) Inventor: MCNULTY, Amy K., Saint Paul, Minnesota 55133-3427 (US); FITZSIMONS, Robert T. Jr., Saint Paul, Minnesota 55133-3427 (US); HALVERSON, Kurt J., Saint Paul, Minnesota 55133-3427 (US); BAKER, Bryan A., Saint Paul, Minnesota 55133-3427 (US); NINKOVIC, Jana, Saint Paul, Minnesota 55133-3427 (US); LIU, Jie, Saint Paul, Minnesota 55133-3427 (US); DAI, Minghua, Saint Paul, Minnesota 55133-3427 (US); ZHANG, Wei, Saint Paul, Minnesota 55133-3427 (US); SGOLASTRA, Federica, Saint Paul, Minnesota 55133-3427 (US)
(74) Representative: Mathys & Squire
(86) International application number: PCT/US2017/038939
(87) International publication number: WO 2018/005275

(56) References cited:
- WO-A1-2014/179188
- US-A1- 2010 160 876
- US-A1- 2013 165 836

## Description

### BACKGROUND

Clinical studies and practice have shown that providing a negative pressure in proximity to a tissue site promotes the growth of new tissues at the tissue site. The application of negative pressure is successful in treating wounds. This treatment (frequently referred to in the medical community as "negative pressure wound therapy (NPWT)," "reduced pressure therapy," or "vacuum therapy") provides a number of benefits, including faster healing and increased formulation of granulation tissue. Typically, reduced pressure is applied to tissues through a foam, a pad or other manifolding device, such as gauze. The manifolding device typically contains cells, pores or other openings that are capable of distributing reduced pressure to the tissue and channeling fluids that are drawn from the tissue. The porous pad often is incorporated into a dressing having other components that facilitate treatment.

US 2010/160876 A1 describes a reduced-pressure wound treatment system for treating a tissue site on a patient, the system comprising: a manifold structure for disposing proximate the tissue site, the manifold structure comprising: a plurality of spaced longitudinal members, at least one shaped projection coupled to at least one of the longitudinal members for creating a microstrain at the tissue site, and the at least one shaped projection comprising a columnar member having a distal end and an enlarged member positioned at the distal end of the columnar member, wherein the columnar member has a first outer diameter, and wherein the enlarged member has a second outer diameter, and wherein the second outer diameter of the enlarged member is greater than the first outer diameter of the columnar member; a sealing member for placing over the tissue and manifold structure and operable to form a fluid seal over the tissue site and the manifold structure; and a reduced-pressure subsystem for delivering reduced pressure to the sealing member.

Other known NPWT articles are discussed in U.S. Patent Nos. 7,494,482; 8,057,447; 8,889,243 and 9,107,989.

### SUMMARY

In one aspect, the present disclosure provides an article as set out in claim 1. The article includes a network of interconnected polymeric strands, wherein each of the interconnected polymeric strands has a first surface adapted to contact a tissue site, wherein at least one of the interconnected polymeric strand has a plurality of protrusions extending from the first surface of the interconnected polymeric strands, wherein at least one of the interconnected polymeric strands is non-linear and wherein the interconnected polymeric strands are oriented in the same direction; and a plurality of openings between adjacent polymeric strands; wherein the article is a negative pressure wound therapy article.

In another aspect, the present disclosure provides a system, including the article of the present disclosure and a reduced pressure source fluidly connected to the opening of the article to deliver the reduced pressure through the opening, between the protrusions, and to the tissue site.

Various aspects and advantages of exemplary embodiments of the present disclosure have been summarized. The above Summary is not intended to describe each illustrated embodiment or every implementation of the present disclosure. Further features and advantages are disclosed in the embodiments that follow. The Drawings and the Detailed Description that follow more particularly exemplify certain embodiments using the principles disclosed herein.

### BRIEF DESCRIPTION OF THE DRAWINGS

The disclosure may be more completely understood in consideration of the following detailed description of various embodiments of the disclosure in connection with the accompanying figures, in which:
FIG. 1 illustrates a article according to an embodiment of the present invention.
FIG. 2 illustrates a top view of an article according to an embodiment of the present invention.
FIG. 3 illustrates the shape of the protrusions of the article according to an embodiment of the present invention.
FIG. 4 illustrates a reduced pressure treatment system according to an embodiment of the present invention.
FIG. 5 is a scanning electron microscope image of the article of Example 2.

While the above-identified drawings, which may not be drawn to scale, set forth various embodiments of the present disclosure, other embodiments are also contemplated, as noted in the Detailed Description. In all cases, this disclosure describes the presently disclosed invention by way of representation of exemplary embodiments and not by express limitations. It should be understood that numerous other modifications and embodiments can be devised by those skilled in the art, which fall within the scope of this disclosure.

### DETAILED DESCRIPTION

Before any embodiments of the present disclosure are explained in detail, it is understood that the invention is not limited in its application to the details of use, construction, and the arrangement of components set forth in the following description. The invention is capable of other embodiments and of being practiced or of being carried out in various ways that will become apparent to a person of ordinary skill in the art upon reading the present disclosure. Also, it is understood that the phraseology and terminology used herein is for the purpose of description and should not be regarded as limiting. The use of "including," "comprising," or "having" and variations thereof herein is meant to encompass the items listed thereafter and equivalents thereof as well as additional items. It is understood that other embodiments may be utilized and structural or logical changes may be made without departing from the scope of the present disclosure.

As used in this Specification, the recitation of numerical ranges by endpoints includes all numbers subsumed within that range (e.g. 1 to 5 includes 1, 1.5, 2, 2.75, 3, 3.8, 4, and 5, and the like).

Unless otherwise indicated, all numbers expressing quantities or ingredients, measurement of properties and so forth used in the Specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The article of the present application is well suited to promote tissue growth at the tissue site yet prevent in-growth of new tissue into the article. The article of the present application can help to deliver a significant portion of microstrain to the wound site by the architecture of the article, for example, the surface morphology of the article and thus may allow for lower pressure settings for NPWT to be used (for example, -75 mmHg vs -125 mmHg). This may allow a longer battery life of the NPWT system and the use of smaller pumps for the NPWT.

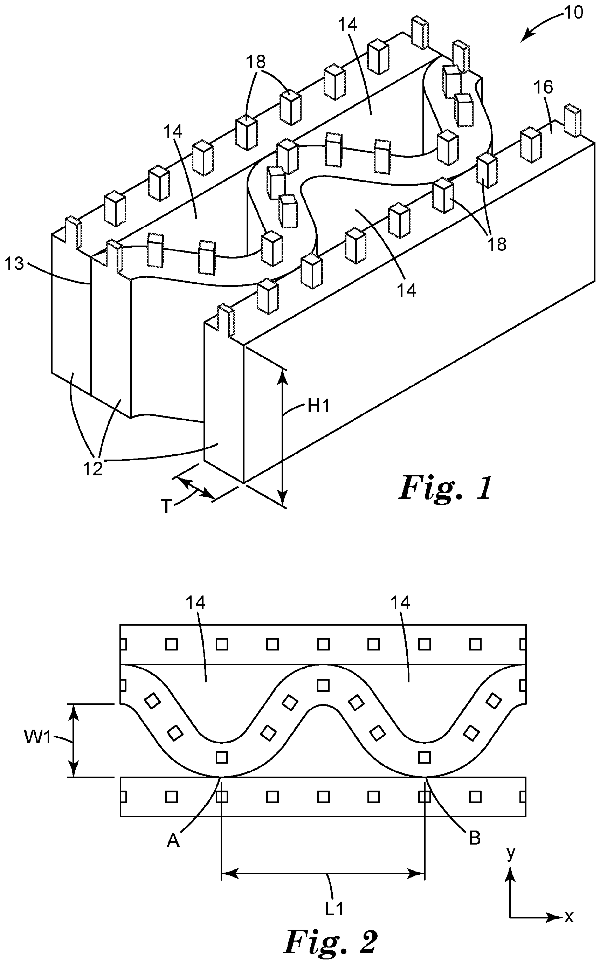

Referring to FIG. 1, an article according to an embodiment of the present invention includes a network of interconnected polymeric strands, or sheets 12 and a plurality of openings 14 between adjacent polymeric strands. Polymeric strands 12 can be connected at connections 13. Typically, there are a plurality of connections 13 between adjacent strands. Polymeric strands 12 have a tissue contact surface 16 as a first surface. The first surface 16 may include a plurality of features, or protrusions 18 that extend from the first surface 16. In some embodiments, the protrusions 18 do not substantially contact each other (i.e., at least 50 (at least 55, 60, 65, 70, 75, 80, 85, 90, 95, 99, or even 100) percent by number do not contact each other). The openings 14 form or provide open fluid channels from the first surface 16 of the network of polymeric strands to a second surface opposite first surface 16. Through the open fluid channels, openings 14 are typically used to allow reduced pressure applied to a tissue site.

Referring more specifically to FIG. 1, the height, H1, of each polymeric strand 12 may be up to 2000 micrometers, up to 1500 micrometers, up to 1000 micrometers, up to 500 micrometers, or up to 400 micrometers. In some embodiments, the height, H1, of each polymeric strand 12 may be no less than 100 micrometers, no less than 200 micrometers, or no less than 300 micrometers. In some embodiments, the height, H1, of each polymeric strand 12 may be between 100 and 2000 micrometers, between 200 and 1500 micrometers, between 300 and 1000 micrometers, between 300 and 500 micrometers or between 300 and 400 micrometers. In some embodiments, the thickness, T, of each polymeric strand 12 may have an average width up to 500 micrometers, up to 400 micrometers, or up to 250 micrometers. In some embodiments, the thickness, T, of each polymeric strand 12 may have an average width no less than 10 micrometers. In some embodiments, the thickness, T, of each polymeric strand 12 may have an average width in a range from 10 micrometers to 500 micrometers, from 10 micrometers to 400 micrometers, or 10 micrometers to 250 micrometers. In some embodiments, the article comprising interconnected polymeric strands has an average thickness not greater than 5 mm. In one embodiment of the present invention, the height and thickness of the interconnected polymeric strands 12 is uniform for a particular article 10. In other embodiments, the height and thickness of the interconnected polymeric strands 12 may be different. For example, the interconnected polymeric strands 12 having different height. Similarly, thickness of the interconnected polymeric strands 12 may vary. In some, embodiments, the interconnected polymeric strands 12 may have a range of thicknesses, for example, the interconnected polymeric strands 12 tends to be thinnest where it abuts an opening.

In some embodiments, the article comprising interconnected polymeric strands has a thickness up to 2 mm, up to 1 mm, up to 500 micrometers, up to 250 micrometers, up to 100 micrometers, up to 75 micrometers, up to 50 micrometers, or up to 25 micrometers. In some embodiments, the article comprising interconnected polymeric strands has a thickness no less than 10 micrometers. In some embodiments, the article comprising interconnected polymeric strands has a thickness in a range from 10 micrometers to 2 mm, 10 micrometers to 1 mm, 10 micrometers to 750 micrometers, 10 micrometers to 500 micrometers, 10 micrometers to 250 micrometers, 10 micrometers to 100 micrometers, 10 micrometers to 75 micrometers, 10 micrometers to 50 micrometers, or 10 micrometers to 25 micrometers. In some embodiments, the article comprising interconnected polymeric strands has an average thickness in a range from 250 micrometers to 5 mm.

In some embodiments, at least one of the interconnected polymeric strands 12 may be non-linear. In some embodiments, at least 25% of the interconnected polymeric strands 12 may be non-linear. In some embodiments, at least 50% of the interconnected polymeric strands 12 may be non-linear. In some embodiments, at least 75% of the interconnected polymeric strands 12 may be non-linear. In some embodiments, essentially all the interconnected polymeric strands 12 may be non-linear. In some embodiments, all of the interconnected polymeric strands 12 may be non-linear. In some embodiments, the non-linear polymeric strand may have a shape of a curve. In some embodiments, the non-linear polymeric strand may have a shape of a sinusoidal curve. In some embodiments, the non-linear polymeric strand may have a shape of a sinusoidal curve. In other embodiments, at least one of the interconnected polymeric strands 12 may be linear. In some other embodiments, 25% to 75% of the interconnected polymeric strands 12 may be linear. In some other embodiments, 50% to 75% of the interconnected polymeric strands 12 may be linear. In certain embodiments, the network of interconnected polymeric strands may include alternating non-linear polymeric strands and linear polymeric strands, as shown in FIG. 2. The interconnected polymeric strands 12 are oriented in the same direction, for example, x direction as illustrated in FIG. 2. In some embodiments, the interconnected polymeric strands 12 do not substantially cross over each other (i.e., at least 50 (at least 55, 60, 65, 70, 75, 80, 85, 90, 95, 99, or even 100) percent by number do not cross over each other).

In some embodiments, aspect ratio (a ratio of the length to the width) of the openings 14 may be greater than 1:1, 1.5:1, 2:1, 3:1 or 5:1. In some embodiments, aspect ratio (a ratio of the length to the width) of the openings 14 may be in a range from 1:1 to 100:1, 1:1 to 75:1, 1:1 to 50:1, 1:1 to 25:1, 2:1 to 100:1 2:1 to 75:1, 2:1 to 50:1, 2:1 to 25:1, or 2:1 to 10:1. The length, L1, of an opening 14 illustrated in FIG. 2 is the longest lateral distance parallel to x direction, for example, the length between connections A and C. If the non-linear polymeric strand has a shape of a sinusoidal curve, the length of the opening 14 equals the wavelength of the sinusoidal curve. The width, W1, of an opening 14 illustrated in FIG. 2 is the longest distance parallel to y direction. If the non-linear polymeric strand has a shape of a sinusoidal curve, the width of the opening 14 equals two times amplification of the sinusoidal curve. Openings 14 of the article may have a range of L1 and W1 values as a result in part of variable spacing of the connections A and B. In some embodiments, the openings have widths, W1, up to 10 mm, up to 1 mm or up to 0.5 mm. In some embodiments, the openings have widths, W1, at least 5 micrometers or at least 10 micrometers. In some embodiments, the openings have widths, W1, in a range from 5 micrometers to 1 mm or from 10 micrometers to 0.5 mm. In some embodiments, the openings have lengths, L1, up to 10 mm or up to 1 mm. In some embodiments, the openings have lengths, L1, at least 100 micrometers. In some embodiments, the openings have lengths, L1, in a range from 100 micrometers to 10 mm or from 100 micrometers to 1 mm. FIGs 1 and 2 are idealized illustrations of one embodiment of the present application. In some embodiments, the openings 14 may have irregularly formed perimeters. This can mean that the openings have irregular shapes (that is, no lines of symmetry). They may have edges that are not smooth (e.g., jagged or feathery edges). Irregularly formed openings can also have a variety of thicknesses of the polymeric strands surrounding the openings.

In some embodiments, openings 14 may have any suitable shape, for example, a shape selected from shapes of ellipse, oval, pointed oval (or lens), diamond, ½ ellipse, ½ oval, ½ lens, triangle, etc. In some embodiments, the openings of the mechanical fastening nets described herein have at least two pointed ends. In some embodiments, at least some of the openings are elongated with two pointed ends. In some embodiments, at least some of the openings are elongated with two opposed pointed ends. In some embodiments, at least some of the openings are ovals.

In some embodiments, the article described herein have a total open area for each of the first and second, generally opposed surfaces of not greater than 50 (in some embodiments, not greater than 45, 40, 35, 30, 25, 20. 15, 10, 5, 4 3, 2, 1, 0.75, 0.5, 0.25, or even not greater than 0.1) percent of the total area of the respective surface. In some embodiments, for at least a majority of the openings of the article described herein, the maximum area of each opening is not greater than is 5 (in some embodiments, not greater than 2.5, 2, 1, 0.5, 0.1, 0.05, 0.01, 0.075, or even not greater than 0.005)mm². Individual openings range from 0.005 mm² to 5 mm². In some embodiments, the article according to the present disclosure have in a range from 50,000 to 6,000,000 (in some embodiments, 100,000 to 6,000,000, 500,000 to, 6,000,000, or even 1,000,000 to 6,000,000) openings/m².

In some embodiments, the tensile strength of the article parallel to x direction is greater than the tensile strength of the article parallel to y direction. Therefore, the article is easier to be stretched in y direction than in x direction. In some embodiments, the tensile strength of the article parallel to x direction is at least 2.23 Mpa, at least 2.25 Mpa, at least 2.5 Mpa or at least 3.0 Mpa. In some embodiments, the tensile strength of the article parallel to x direction is up to 5.42 MPa, up to 5.3 MPa, up to 5.0 MPa, or up to 4.5 MPa. In some embodiments, the tensile strength of the article parallel to x direction is from 2.23 Mpa to 5.42 MPa., from 2.5 Mpa to 5.0 MPa or from 3.0 Mpa to 4.5 MPa. The Young's modulus of the article is up to 10.6 MPa, up to 10.0 MPa, up to 9.0 MPa, or up to 8.0 MPa. The Young's modulus of the article is at least 3.85 MPa, at least 4.0 MPa has a range from 3.85 MPa to 10.6 MPa in a direction parallel to x direction.

The shape, sizing, and spacing of the protrusions 18 may vary depending upon the particular tissue site being treated, the type of material from which the protrusions 18 and polymeric strands are made, and the amount of reduced pressure being applied to the tissue site. For example, for tissue sites that are highly exudating, it may be advantageous to position the protrusions farther apart or reduce the density of protrusions on the first surface to maintain adequate distribution channels between the protrusions 18. In one embodiment of the present invention, the shape, sizing and spacing of the protrusions 18 is uniform for a particular article 10. In other embodiments, the shape, sizing, and spacing of the protrusions 18 may be different. For example, protrusions 18 having different cross-sectional shapes may be disposed on the first surface. Similarly, the sizing and spacing of the protrusions 18 may vary to supply selected portions of the tissue site with different (more or less) reduced pressure and different flow rate for exudates withdrawn.

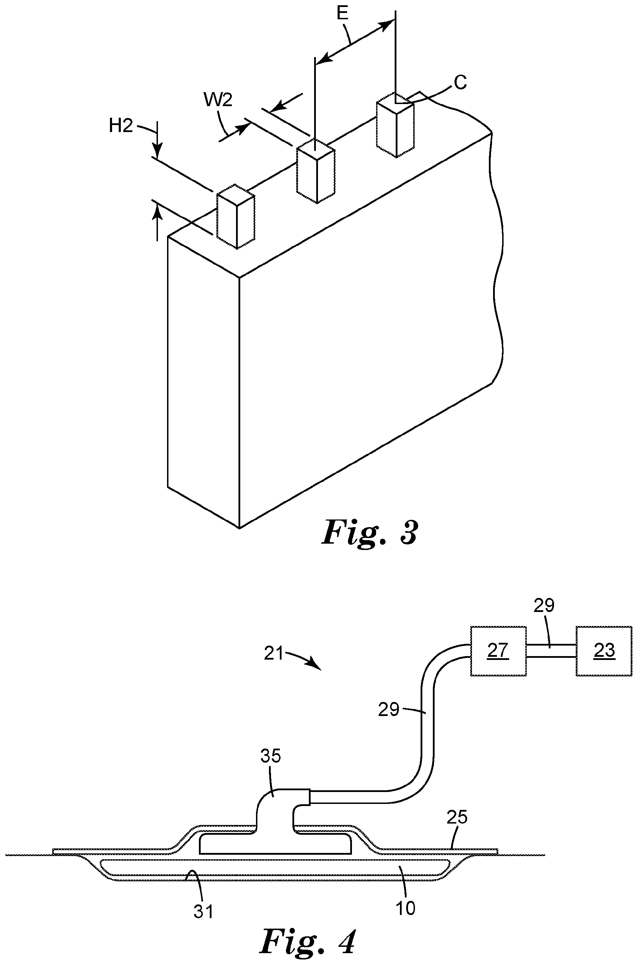

Referring more specifically to FIG. 3, the height, H2, of the protrusions 18 may be up to 1000 micrometers, up to 500 micrometers or up to 450 micrometers. In some embodiments, the height, H2, of the protrusions 18 may be at least 100 micrometers or at least 200 micrometers. In some embodiments, the height, H2, of the protrusions 18 may be between 100 and 1000 micrometers, between 200 and 500 micrometers or between 200 and 450 micrometers. The width, W2, of each protrusion may be up to 1000 micrometers, up to 900 micrometers, up to 800 micrometers, up to 700 micrometers or up to 600 micrometers. In some embodiments, the width, W2, of each protrusion may be at least 10 micrometers, at least 100 micrometers, at least 200 micrometers, at least 300 micrometers or at least 400 micrometers. In some embodiments, the width, W2, of each protrusion may be between 10 and 1000 micrometers, between 100 and 900 micrometers, between 200 and 800 micrometers, between 300 and 700 micrometers, or between 400 and 600 micrometers. In some embodiments, the width, W2, of each protrusion may be 500 micrometers. The width of the protrusions 18 illustrated in FIG. 3 is an edge length of the square since the cross-sectional shape of each protrusions 18 is square. If the protrusions 18 are circular in cross-sectional shape, the width of the protrusions 18 equals the diameter since the cross-sectional shape of each protrusion 18 is circular. For other cross-sectional shapes, the width is the average of the longest lateral distance through the centroid, C, of the cross section and the shortest lateral distance through the centroid of the cross section. It is generally preferred that the height of the protrusions 18 be no more than the width of the protrusions 18. More specifically, the ratio of height to width, H2:W2 of the protrusions 18, should be no more than 1:1. When the ratio of height to width, H2:W2 of the protrusions 18, is more than 1:1, the protrusions 18 is more prone to fall over the openings 14, thus reducing the fluid flow through the openings 14. The lateral, center-to-center spacing, E, between each protrusion 18 may be between 0.1 and 2.0 millimeters, between 0.5 and 1.5 millimeters or between 0.7 and 1.3 millimeters. The spacing of the protrusions 18 create distribution channels through which reduced pressure may be delivered to the tissue site and exudates withdrawn from the tissue site. The density of protrusions on the first surface may be less than 1,000/square inch to facilitate reduced pressure delivered to the tissue site and exudates withdrawn from the tissue site. In some embodiments, the density of protrusions on the first surface may be less than 1,000/square inch, less than 900/square inch, less than 800/square inch, less than 700/square inch, less than 600/square inch, or less than 500/square inch. In some embodiments, the number of protrusions in the article can be greater than the number of openings. For example, the ratio of the number of protrusions to the number of openings can be more than 1, 1.5, 2, 2.5, 3, 4, 5, or 10. In some embodiments, the article of the present disclosure can be a mechanical fastening net or a mechanical fastening sheet with protrusions.

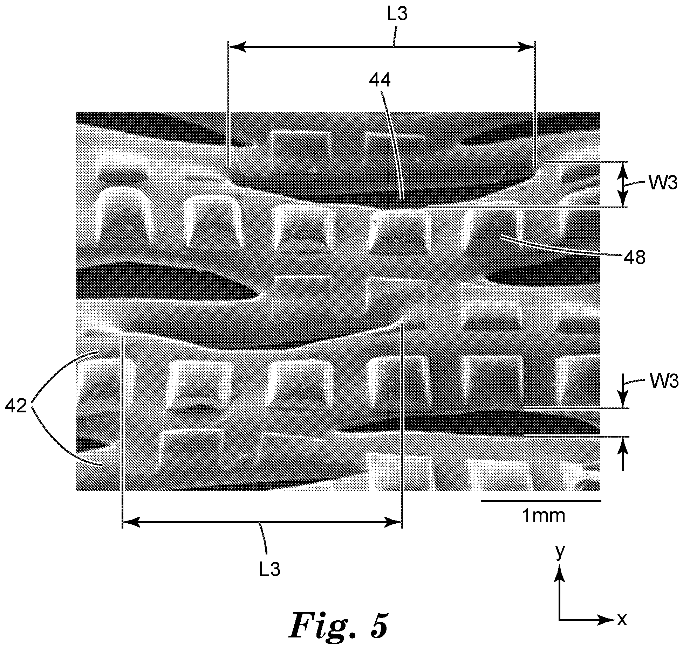

In some embodiments, protrusions 18 are oriented along the interconnected polymeric strands 12 as illustrated in FIG. 1. In other embodiments, protrusions 48 are oriented in substantially same orientation, for example x direction, as illustrated in FIG. 5.

The presence and sizing of the protrusions 18 allow the protrusions 18 to distribute reduced pressure to the tissue site, but prevent new tissue that grows at the tissue site from attaching to the protrusions 18 or growing into the spacing between protrusions 18. While new tissue growth may wrap around some of the protrusions 18, the new tissue is not capable of securing itself to the protrusions 18 since the base of each protrusions is anchored to the first surface 16.

In addition to distributing reduced pressure to the tissue site, the article 10 also serves to impart stresses and strains to the tissue site similar to those seen with cellular foam that traditionally has been used in reduced pressure systems. Other materials sometimes used in reduced pressure systems, such as gauze, do not have this effect on tissue. Unbound by the theory, the stresses and strains created by the article 10 are believed to cause micro-deformation of existing tissues and plays a significant role in the generation of new tissues at the tissue site. The amount of stress and strain imparted to a tissue site is determined by the amount of reduced pressure supplied to the tissue site and the surface morphology of the article that contacts the tissue site. As reduced pressure is applied, portions of the tissue site are pulled against the article 10, and more particularly against the protrusions 18, which results in the development of stresses and strains within the tissue. In some embodiments, the article of the present disclosure can be a mechanical fastening net or a mechanical fastening sheet with protrusions.

Referring to FIG. 4, a reduced pressure treatment system 21 according to an embodiment of the present invention includes a reduced pressure dressing, or article 10 fluidly connected to a reduced pressure conduit 29. The reduced pressure conduit 29 is fluidly connected to a reduced pressure source 23 such as a vacuum pump or another source of suction. The article 10 is placed against a tissue site 31 of a patient and is used to distribute a reduced pressure provided by the reduced pressure source 23. Typically, reduced pressure is maintained at the tissue site by placing an impermeable or semi-permeable cover 25 over the article 10 and the tissue site 31. The reduced pressure also serves to draw wound exudates and other fluids from the tissue site 31. A canister 27 may be fluidly connected to the reduced pressure conduit 29 and disposed between the article 10 and the reduced pressure source 23 to collect the fluids drawn from the tissue site 31. A distribution adapter 35 may be connected to the reduced pressure conduit 29 and positioned on the article 10 to aid in distributing the reduced pressure to the distribution manifold 10.

The article 10 may further include a cellular foam or another material that is positioned adjacent to or attached to the surface of the interconnected polymeric strands opposite the protrusions 18. The use of a cellular foam or other material increases the ability of the reduced pressure conduit 29 or the distribution adapter 35 to deliver and distribute reduced pressure to the article 10. The protrusions 18 and interconnected polymeric strands serve as a barrier to new tissue growth entering pores of the cellular foam or other material.

In some embodiments, the interconnected polymeric strands 12 can include an elastomeric polymer. Elastomeric polymer can be any suitable elastomeric polymer, including but not limited to polyolefins and polyurethanes. In some embodiments, elastomeric polymer can be a medical grade material that is relatively impermeable to fluid flow. Alternatively, elastomeric polymer can be a semi-permeable material that allows select fluids or amounts of fluids to pass. In some embodiments, the interconnected polymeric strands 12 are formed from the same material as the protrusions 18. In some embodiments, the interconnected polymeric strands 12 can be formed from a different material as the protrusions 18. In some embodiments, the composition of interconnected polymeric strands 12 may be formed from different materials.

Some embodiments of the present wound-treatment methods can include positioning the article of present disclosure on a wound of a patient and applying a reduced pressure to the wound through the article (e.g., through the openings). Some embodiments further comprise: coupling a drape to skin adjacent the wound such that the drape covers the article and the wound, and forms a space between the drape and the wound. In some embodiments, positioning the article on the wound can include placing the article over the wound with the protrusions on the first surface facing the wound. In some embodiments, applying the reduced pressure to the wound comprises activating a vacuum source (e.g., reduced pressure source 23 of FIG. 4) that is coupled to the article. Some embodiments comprise: delivering a fluid to the wound through the article. In some embodiments, delivering a fluid comprises activating a fluid source that is coupled to the article.

The following working examples are intended to be illustrative of the present disclosure and not limiting.

### EXAMPLES

### Example 1.

A polyolefin net was prepared using ENGAGE 8200 polyolefin elastomer (obtained from the Dow Chemical Company, Midland, MI) according to the methods described in United States Patent Application 2014/0234606 (Ausen), herein incorporated by reference in its entirety. The resulting net material had strand width ranges of about 0.42-0.83 mm, pore width ranges of about 0.15-0.57 mm, pore length ranges of about 1.5-2.1 mm, and a thickness range of about 0.75-1.24 mm. The net sample was embossed by stacking from top to bottom a steel plate, a square pattern polypropylene film that served as the mold (inner dimension of a square being approximately 0.5 mm), the polyolefin net sample, a release liner, and a second steel plate. The stack was placed in a Carver Auto Series NE Automatic Hydraulic Press (Model 3895.4NE1000, Carver Inc., Wabasha, IN) with the bottom platen of the press set at 21 °C and the top platen set at 121 °C. The press was closed and the sample was held at a set force of 794 kg for two minutes, followed by a cool down period of four minutes to 65.5 °C. The press was opened and the embossed sample was removed from the press. The resulting embossed article had a base thickness range of about 0.56-0.65 mm; strand width ranges of about 0.45-0.50 mm; openings with width ranges of about 0.13-0.37 mm and length ranges of about 0.87-1.10 mm; protrusions with width at base of about 0.5 mm and height ranges of about 0.29-0.50 mm; and protrusion spacing ranges of about 0.77-0.94 mm.

### Example 2.

A polyurethane net was prepared using IROGRAN A 60 E 4902 thermoplastic polyurethane (obtained from the Huntsman Corporation, The Woodlands, TX) according to the methods described in United States Patent Application 2014/0234606 (Ausen). The resulting net material had strand width ranges of about 0.29-0.46 mm, pore width ranges of about 0.20-0.37 mm, pore length ranges of about 1.8-3.0 mm, and a thickness range of about 0.66-0.73 mm. The net sample was embossed by stacking from top to bottom a steel plate, a square pattern polypropylene film that served as the mold (inner dimension of a square being approximately 0.5 mm), the polyolefin net sample, a release liner, and a second steel plate. The stack was placed in a Carver Auto Series NE Automatic Hydraulic Press (Model 3895.4NE1000, Carver Inc.) with the bottom platen of the press set at 176.6 °C and the top platen set at 21 °C. The stack was warmed on the apparatus for two minutes. The press was then closed and the sample was held at a set force of 907 kg for two minutes, followed by a cool down period of five minutes to 93.3 °C. The press was opened and the embossed sample was removed from the press. The resulting embossed article had a base thickness range of about 0.36-0.49 mm; strand width ranges of about 0.33-1.10 mm; openings with width ranges of about 0.25-0.58 mm and lengths of about 2.20 mm; protrusions with width at base of about 0.5 mm and height ranges of about 0.27-0.42 mm; and protrusion spacing ranges of about 0.73-0.88 mm. A scanning electron microscope image of the article is shown in FIG. 5. The image shows a network of interconnected polymeric strands 42 and a plurality of openings 44 between adjacent polymeric strands. Polymeric strands 42 have a plurality of features, or protrusions 48 on a surface of polymeric strands. The exemplary length, L3 and width, W3, of opening 44 is illustrated in FIG. 5. The interconnected polymeric strands 42 are oriented in x direction as illustrated in FIG. 5.

### Example 3. Determination of Fibroblast Proliferation using an In Vitro Cell Culture Device.

The cell culture device having a lower base unit, a seal, an upper base unit, a cell culture insert for growing cell cultures, a sealing member, a guide tube, a support bracket, a vacuum conduit, a media conduit, a second seal and screw, was used. Fibroblasts were encapsulated in a fibrin gel matrix (clot) to simulate a component of the wound healing environment. The matrix was prepared by the following three step procedure. First, a layer of fibrin gel was prepared and applied to the internal surface of the permeable membrane (24 mm diameter with 1 micron pore size) in a MILLICELL hanging cell culture insert (obtained from EMD Millipore, Billerica, MA) by combining 1 mL of human fibrinogen (concentration of 9.8 mg per mL of porcine plasma) with 0.25 mL of thrombin (concentration of 500-1100 units per mL of porcine plasma) (human fibrinogen obtained from Sigma-Aldrich Corporation, St. Louis, MO; thrombin obtained from BioPharm Laboratories, Bluffdale, UT; porcine plasma obtained from Lampire Biological Lab, Piphersville, PA). This layer was covered with a layer of about 50,000 fibroblasts (obtained from Invitrogen Corporation, Carlsbad, CA). The fibroblast layer was then covered with a third (or top layer) of fibrin gel prepared in the same manner as for the first layer. Following encapsulation in the gel, the fibroblasts were grown in an incubator at 37 °C for two days.

In the cell proliferation assay, the article of Example 1 was placed over the top layer of the matrix with the surface of the article containing the protrusion elements facing and in contact with the gel matrix. The opposite face of the article was covered with an 10 mm thick pad of GRANUFOAM polyurethane foam (V.A.C. Granufoam Dressing Medium, KCI Inc., San Antonio, TX). The combined article of Example 1 and GRANUFOAM cover pad formed the test dressing. Fibroblast Culture Medium 106 (obtained from Invitrogen Corporation) was continuously supplied to the gel matrix by means of a peristaltic pump. Negative pressure (-125 mm Hg) was applied to the device for 48 hours at 37 °C. The device was then dismantled and the fibroblast sample was evaluated for cell proliferation using an XTT colorimetric assay kit (obtained from Invitrogen Corporation) with the absorbance measurements taken at 570 nm using a SpectraMax M5 plate reader (Molecular Devices, Sunnyvale, CA). The level of fibroblast cell proliferation for the example was compared to a control experiment. In the control experiment, the same procedure was used except no test dressing was added to the apparatus and negative pressure was not applied for the 48 hour test period. In Table 1, the mean percent increase in recorded absorbance for the example compared to the control is reported (3 replicates).

### Comparative Example A.

The same procedure as described as in Example 3 with the exception that a 10 mm thick multilayer sheet of gauze (non-stick gauze pads were obtained from Johnson and Johnson Company, New Brunswick, NJ) was used as the test dressing. In Tables 1 and 2, the mean percent increase in recorded absorbance for Comparative Example A compared to the control is reported (3 replicates with Tables 1 and 2 being separate experiments).

**Table 1.**

| Example | Percent Increase in Absorbance as Compared to Control |
|---|---|
| Example 3 | 6.0 |
| Comparative Example A | 3.8 |

### Example 4.

The same procedure as described as in Example 3 was used with the exception that the article of Example 2 was combined with a GRANUFOAM pad to form the test dressing. The mean percent increase in recorded absorbance for Example 4 is reported (3 replicates) in Table 2.

### Comparative Example B.

The same procedure as described as in Example 3 was used with the exception that the non-embossed net used to prepare the article of Example 2 (instead of the embossed article) was combined with a GRANUFOAM pad to form the test dressing. The mean percent increase in recorded absorbance for Comparative Example B compared to the control is reported (3 replicates) in Table 2.

**Table 2.**

| Example | Percent Increase in Absorbance as Compared to Control |
|---|---|
| Example 4 | 10.3 |
| Comparative Example A | 3.6 |
| Comparative Example B | 8.4 |

### Example 5.

The polyolefin net described in Example 1 sample was embossed by stacking from top to bottom a steel plate, a square pattern polypropylene film that served as the mold (inner dimension of a square being approximately 0.5 mm), the polyolefin net sample, a release liner, and a second steel plate. The stack was placed in a Carver Auto Series NE Automatic Hydraulic Press (Model 3895.4NE1000, Carver Inc.) with the bottom platen of the press set at 21 °C and the top platen set at 107 °C. The press was closed and the sample was held at a set force of 794 kg for two minutes, followed by a cool down period of two minutes to 65.5 °C. The press was opened and the embossed sample was removed from the press. The resulting embossed article had a base thickness range of about 0.36-0.99 mm; strand width ranges of about 0.27-1.3 mm; openings with width ranges of about 0.10-1.10 mm and length ranges of about 2.50-2.70 mm; protrusions with width at base of about 0.5 mm and height ranges of about 0.19-0.22 mm; and protrusion spacing ranges of about 0.70-0.77 mm.

### Example 6.

The polyurethane net described in Example 2 was embossed by stacking from top to bottom a steel plate, a square pattern polypropylene film that served as the mold (inner dimension of a square being approximately 0.5 mm), the polyolefin net sample, a release liner, and a second steel plate. The stack was placed in a Carver Auto Series NE Automatic Hydraulic Press (Model 3895.4NE1000, Carver Inc.) with the bottom platen of the press set at 176.6 °C and the top platen set at 21 °C. The stack was warmed on the apparatus for two minutes. The press was then closed and the sample was held at a set force of 907 kg for four minutes, followed by a cool down period of three minutes to 93.3 °C. The press was opened and the embossed sample was removed from the press. The resulting embossed article had a base thickness range of about 0.41-0.52 mm; strand width ranges of about 0.37-0.96 mm; openings with width ranges of about 0.13-0.46 mm and length ranges of about 0.92-1.60 mm; protrusions with width at base of about 0.5 mm and height ranges of about 0.21-0.40 mm; and protrusion spacing ranges of about 0.77-0.86 mm.

### Example 7. Mechanical Property Tests

The mechanical properties of the embossed articles of Example 5 and Example 6 were determined by testing individual embossed article samples with an INSTRON Model 5943 Mechanical Testing Apparatus (INSTRON Corporation, Norwood, MA). The tests were conducted at ambient conditions using a 100 N load cell. Samples were held with pneumatic grips. The rate of grip separation was 50 mm/minute. Measurements of samples in the direction of the interconnected strands (x-direction of FIG 2) were conducted using a dog-bone punch pattern with an approximately 6 mm width at center. Measurements of samples in the cross-direction from the direction of the interconnected strands (y-direction of FIG 2) were conducted using a rectangular block pattern (25 mm by 75-100 mm). Sample dimensions were measured for each test sample in order to calculate cross-sectional area. The mean measured values of ultimate tensile strength (MPa), Young's modulus (MPa), and percent strain are reported in Table 3.

**Table 3.**

| Article of Example | Number of Replicates | Test Direction | Ultimate Tensile Strength (MPa) | Young's Modulus (MPa) | Percent Strain |
|---|---|---|---|---|---|
| 5 | 2 | x-direction | 2.38 | 4.63 | 212.9 |
| | 3 | y-direction | 0.34 | 0.36 | 119.8 |
| 6 | 1 | x-direction | 2.02 | 2.86 | 226.2 |
| | 3 | y-direction | 0.16 | 1.37 | 24.5 |

Illustrative embodiments of this invention are discussed and reference has been made to possible variations within the scope of this invention. For example, features depicted in connection with one illustrative embodiment may be used in connection with other embodiments of the invention. These and other variations and modifications in the invention will be apparent to those skilled in the art without departing from the scope of the invention, and it should be understood that this invention is not limited to the illustrative embodiments set forth herein. Accordingly, the invention is to be limited only by the claims provided below.

## Claims

1. An article (10), comprising:
a network of interconnected polymeric strands (12), wherein each of the interconnected polymeric strands has a first surface (16) adapted to contact a tissue site, wherein at least one of the interconnected polymeric strands (12) has a plurality of protrusions (18) extending from the first surface (16) of the interconnected polymeric strands (12), wherein at least one of the interconnected polymeric strands is non-linear, and wherein the interconnected polymeric strands are oriented in the same direction; and
a plurality of openings (14) between adjacent interconnected polymeric strands (12);
wherein the article is a negative pressure wound therapy article.

2. The article of claim 1, wherein at least one of the interconnected polymeric strands is linear.

3. The article of any one of claims 1 to 2, wherein the network comprises alternating non-linear polymeric strands and linear polymeric strands.

4. The article of any one of claims 1 to 3, wherein the non-linear polymeric strand has a sinusoidal curve.

5. The article of any one of claims 1 to 4, wherein the polymeric strands comprise an elastomeric polymer.

6. The article of claim 5, wherein the elastomeric polymer is selected from polyolefins or polyurethanes.

7. The article of any one of claims 1 to 6, wherein the protrusions have a height of 100 µm to 1000 µm.

8. The article of any one of claims 1 to 7, wherein the protrusions have a width of 10 µm to 1000 µm.

9. The article of any one of claims 1 to 8, wherein a ratio of the height to width of the protrusions is no more than 1: 1

10. The article of any one of claims 1 to 9, wherein the density of protrusions extending from the first surface is less than 155 protrusions/cm² (1,000/square inch).

11. The article of any one of claims 1 to 10, wherein aspect ratio of the openings is greater than 1:1.

12. The article of any one of claims 1 to 11, wherein essentially all the interconnected polymeric strands are non-linear.

13. A system, comprising:
the article of any one of claims 1 to 12; and
a reduced pressure source fluidly connected to the opening of the article to deliver the reduced pressure through the opening, between the protrusions, and to the tissue site.

## Patentansprüche

1. Ein Artikel (10), aufweisend:
ein Netzwerk von miteinander verbundenen Polymersträngen (12), wobei jeder der miteinander verbundenen Polymerstränge eine erste Oberfläche (16) aufweist, die angepasst ist, um eine Gewebestelle zu kontaktieren, wobei mindestens einer der miteinander verbundenen Polymerstränge (12) eine Vielzahl von Vorsprüngen (18) aufweist, die sich von der ersten Oberfläche (16) der miteinander verbundenen Polymerstränge (12) erstrecken, wobei mindestens einer der miteinander verbundenen Polymerstränge nicht linear ist und wobei die miteinander verbundenen Polymerstränge in der gleichen Richtung ausgerichtet sind; und
eine Vielzahl von Öffnungen (14) zwischen angrenzenden, miteinander verbundenen Polymersträngen (12);
wobei der Artikel ein Unterdruck-Wundtherapieartikel ist.

2. Der Artikel nach Anspruch 1, wobei mindestens einer der miteinander verbundenen Polymerstränge linear ist.

3. Der Artikel nach einem der Ansprüche 1 bis 2, wobei das Netzwerk abwechselnd nicht lineare Polymerstränge und lineare Polymerstränge aufweist.

4. Der Artikel nach einem der Ansprüche 1 bis 3, wobei der nicht lineare Polymerstrang eine sinusförmige Kurve aufweist.

5. Der Artikel nach einem der Ansprüche 1 bis 4, wobei die Polymerstränge ein elastomeres Polymer aufweisen.

6. Der Artikel nach Anspruch 5, wobei das elastomere Polymer aus Polyolefinen oder Polyurethanen ausgewählt ist.

7. Der Artikel nach einem der Ansprüche 1 bis 6, wobei die Vorsprünge eine Höhe von 100 µm bis 1000 µm aufweisen.

8. Der Artikel nach einem der Ansprüche 1 bis 7, wobei die Vorsprünge eine Breite von 10 µm bis 1000 µm aufweisen.

9. Der Artikel nach einem der Ansprüche 1 bis 8, wobei ein Verhältnis von der Höhe zu der Breite der Vorsprünge nicht mehr als 1:1 beträgt

10. Der Artikel nach einem der Ansprüche 1 bis 9, wobei die Dichte der Vorsprünge, die sich von der ersten Oberfläche erstrecken, weniger als 155 Vorsprünge/cm² (1.000/Quadratzoll) beträgt.

11. Der Artikel nach einem der Ansprüche 1 bis 10, wobei ein Seitenverhältnis der Öffnungen größer als 1:1 ist.

12. Der Artikel nach einem der Ansprüche 1 bis 11, wobei im Wesentlichen sämtliche der miteinander verbundenen Polymerstränge nicht linear sind.

13. Ein System, aufweisend:
den Artikel nach einem der Ansprüche 1 bis 12; und
eine Unterdruckquelle, die mit der Öffnung des Artikels in Fluidverbindung steht, um den Unterdruck durch die Öffnung, zwischen den Vorsprüngen und an der Gewebestelle abzugeben.

## Revendications

1. Article (10) comprenant :
un réseau de brins polymères interconnectés (12), dans lequel chacun des brins polymères interconnectés a une première surface (16) adaptée pour entrer en contact avec un site tissulaire, dans lequel au moins un des brins polymères interconnectés (12) a une pluralité de protubérances (18) s'étendant à partir de la première surface (16) des brins polymères interconnectés (12), dans lequel au moins un des brins polymères interconnectés n'est pas linéaire, et dans lequel les brins polymères interconnectés sont orientés dans la même direction ; et
une pluralité d'ouvertures (14) entre les brins polymères adjacents interconnectés (12) ;
dans lequel l'article est un article de thérapie des plaies par pression négative.

2. Article selon la revendication 1, dans lequel au moins un des brins polymères interconnectés est linéaire.

3. Article selon l'une des revendications 1 à 2, dans lequel le réseau comprend une alternance de brins polymères non linéaires et de brins polymères linéaires.

4. Article selon l'une des revendications 1 à 3, dans lequel le brin polymère non linéaire a une courbe sinusoïdale.

5. Article selon l'une des revendications 1 à 4, dans lequel les brins polymères comprennent un polymère élastomère.

6. Article selon la revendication 5, dans lequel le polymère élastomère est choisi parmi les polyoléfines ou les polyuréthanes.

7. Article selon l'une des revendications 1 à 6, dans lequel les protubérances ont une hauteur de 100 µm à 1000 µm.

8. Article selon l'une des revendications 1 à 7, dans lequel les protubérances ont une largeur comprise entre 10 µm et 1000 µm.

9. Article selon l'une des revendications 1 à 8, dans lequel le rapport entre la hauteur et la largeur des protubérances n'est pas supérieur à 1:1

10. Article selon l'une des revendications 1 à 9, dans lequel la densité des protubérances s'étendant à partir de la première surface est inférieure à 155 protubérances/cm² (1000/pouce carré).

11. Article selon l'une des revendications 1 à 10, dans lequel le rapport d'aspect des ouvertures est supérieur à 1:1.

12. Article selon l'une des revendications 1 à 11, dans lequel essentiellement tous les brins polymères interconnectés sont non linéaires.

13. Système comprenant :
l'article selon l'une des revendications 1 à 12 ; et
une source de pression réduite reliée fluidiquement à l'ouverture de l'article pour délivrer la pression réduite à travers l'ouverture, entre les protubérances et jusqu'au site tissulaire.
